# EUROPÄISCHE PATENTSCHRIFT

(11) **EP 1 517 668 B1**
(45) Veröffentlichungstag und Bekanntmachung des Hinweises auf die Patenterteilung: **08.10.2008**
(21) Anmeldenummer: 03738068.0
(22) Anmeldetag: 20.06.2003
(51) Int. Cl.: A61K 8/11, A61K 8/02, A61Q 5/10

(54) **VERFAHREN ZUR SCHONENDEN OXIDATIVEN FÄRBUNG VON HAAREN**
GENTLE OXIDATIVE HAIR DYEING METHODS
PROCEDES DE COLORATION OXYDATIVE DELICATE DE CHEVEUX

(30) Priorität: 29.06.2002 DE 10229420
(43) Veröffentlichungstag der Anmeldung: 30.03.2005
(73) Patentinhaber: Henkel AG & Co. KGaA, 40589 Düsseldorf (DE)
(72) Erfinder: KLEEN, Astrid, 22763 Hamburg (DE); HOLLENBERG, Detlef, 40699 Erkrath (DE); HÖFFKES, Horst, 40595 Düsseldorf (DE); BRABÄNDER, Oliver, 46049 Oberhausen (DE)
(86) Internationale Anmeldenummer: PCT/EP2003/006499
(87) Internationale Veröffentlichungsnummer: WO 2004/002439

(56) Entgegenhaltungen:
- EP-A- 1 300 136
- WO-A-01/66068
- WO-A-03/030848
- DE-A- 4 123 941
- DE-A- 19 949 033
- DE-C1- 19 721 797
- US-A- 4 425 132
- US-A- 4 844 711
- US-A- 5 053 051
- US-A- 5 431 698
- US-A- 5 879 412
- US-A- 5 919 273
- US-A- 6 106 579
- US-B1- 6 190 421
- US-B1- 6 379 657

## Beschreibung

Gegenstand der Erfindung ist ein Verfahren zur schonenden oxidativen Färbung von menschlichen Haaren.

Zubereitungen zum Tönen und Färben von Haaren sind ein wichtiger Typ von kosmetischen Mitteln. Sie können dazu dienen, die natürliche Haarfarbe gemäß den Wünschen und Bedürfnissen der entsprechenden Person leicht oder stärker zu nuancieren, eine gänzlich andere Haarfarbe zu erzielen oder unerwünschte Farbtöne, wie beispielsweise Grautöne, zu überdecken. Übliche Haarfärbemittel werden, je nach gewünschter Farbe und Dauerhaftigkeit der Färbung, entweder auf der Basis von Oxidationsfarbstoffen oder auf der Basis von direktziehenden Farbstoffen formuliert. Häufig werden auch Kombinationen von Oxidationsfarbstoffen und direktziehenden Farbstoffen zur Erzielung spezieller Nuancen eingesetzt.

Gute Farbstoffe zeichnen sich durch eine hohe Farbstärke aus. Weiterhin sind gute Schweiß-, Wärme-, Dauerwell-, Wasch- und Lichtechtheit gewünscht. Ferner sollten sie in toxikologischer und dermatologischer Hinsicht unbedenklich sein. Es ist auch von Vorteil, wenn die Substanzen eine hohe Löslichkeit in verschiedenen Basisformulierungen besitzen.

Färbemittel auf der Basis von Oxidationsfarbstoffen führen zu brillanten und dauerhaften Farbtönen. Sie werden jedoch in den heutzutage üblichen Formulierungen in der Regel bei alkalischen pH-Werten von 8 -11 auf das zu färbende Haar aufgetragen und vor oder nach dem Auftragen auf das Haar mit einem chemischen Oxidationsmittel, meist Wasserstoffperoxid, einer Perverbindung oder einem Wasserstoffperoxid-Addukt, aktiviert, d. h. oxidativ zum Farbstoff polymerisiert. Eine solche Behandlung kann, insbesondere bei wiederholter Anwendung oder wenn das Haar bereits durch andere Behandlungen vorgeschädigt ist, eine Schädigung der Haarstruktur zur Folge haben, die sich in einer schlechten Kämmbarkeit und Formbarkeit sowie in einem Verlust an Glanz, Elastizität und Reißfestigkeit äußert. Darüber hinaus kann eine solche Behandlung dem Haar ein insgesamt ungepflegtes und unattraktives Aussehen verleihen.

Die Schädigung des Haares nimmt vom Haaransatz, also von dem Bereich, der der Kopfhaut benachbart ist, bis in die Haarspitze - also mit zunehmendem Alter der Keratinsubstanz - kontinuierlich zu, da der jüngere, der Kopfhaut benachbarte Teil des Haares weniger chemischen, mechanischen und atmosphärischen (UV-Lichteinwirkung) Belastungen ausgesetzt war als die älteren Bereiche bis hin zur Haarspitze. Aus diesem Grund leiden gefärbte Haare auch häufig an unbefriedigender Egalität, da die porösen, geschädigten Partien des Haares viele Farbstoffe stärker adsorbieren und daher tiefer gefärbt werden als der Bereich des Haaransatzes. Gleichzeitig aber wird die Waschfestigkeit im stärker geschädigten Teil des Haares herabgesetzt. Auf diese Weise erscheint das Haar nach mehreren Wäschen im Bereich der Haarspitzen oft stark ausgewaschen.

In der Praxis wird diesen Problemen dadurch entgegengewirkt, dass man zunächst den Haaransatz einfärbt und nach einer gewissen Einwirkungszeit den Farbstoff auch auf die restliche Haarlänge verteilt. Diese Lösung hat sich jedoch als nicht vollkommen zufriedenstellend erwiesen, da einerseits zwar die Einwirkungszeit der alkalischen Oxidationsmittel auf der Haarlänge verkürzt, andererseits jedoch trotzdem bei jeder Färbebehandlung erneut ein oxidativer Angriff auf die bereits geschädigten Haare erfolgt.

In der EP 624 362 B1 wurde vorgeschlagen, zur Schonung der bereits stärker strapazierten Haarlängen in einem zweistufigen Haarfärbeverfahren den Haaransatz mit einem alkalischen und die Haarlängen mit einem sauer eingestellten Oxidationshaarfärbemittel zu färben. Diese Arbeitsweise bedeutet aber für den Haaransatz, dass er mehrfach der Einwirkung des Oxidationsmittels ausgesetzt ist und für die bereits vorgeschädigte Haarlänge, dass sie bei jedem Färbevorgang erneut mit dem Oxidationsmittel in Kontakt kommt.

Die Offenbarung der US 4,425,132 umfasst ebenfalls ein Verfahren zur schonenden Haarfärbung, bei dem das Haar zunächst mit einem alkalischen Oxidationsfärbemittel, und dann in einem zweiten Schritt und ohne weiteren Spülvorgang mit einem neutral bis leicht sauer eingestellten Mittel, enthaltend schonendere, direktziehende Farbstoffe, behandelt wird.

Die WO 01/26616 beschreibt ein zweistufiges Verfahren zum Färben von Haaren, bei dem insbesondere der Haaransatz in der ersten Stufe mit einem üblichen Oxidationsfärbemittel und die Haarlängen in der zweiten Stufe mit einem nicht oxidativen Haarfärbemittel oder einem durch Luftsauerstoff oder katalytisch aktivierten Oxidationshaarfärbemittel gleicher oder ähnlicher Nuance behandelt werden. Auch dieses Verfahren führte zu einer schonenderen Haarfärbung.

Der Offenbarungsgehalt der WO89/06531 beschreibt schließlich die oxidative Färbung von Haaren mit einem alkalisch eingestellten Mittel, das unmittelbar vor der Anwendung durch die Vermischung zweier Komponenten entsteht, die eine enthaltend die Oxidationsfarbstoffvorprodukte und die andere das Oxidationsmittel. Dieses Mittel enthält weiterhin zum Zeitpunkt seiner Entstehung mikroverkapselte, sauer reagierende Wirkstoffe, die sich nach der Mischung auflösen und eine schonende oxidative Haarfärbung bei schwach alkalischen pH-Werten gewährleistet.

DE 41 23 941 A (WELLA AG) offenbart ein Verfahren zur schonenden Färbung und/oder Nachfärbung von menschlichen Haaren (Siehe Anspruch 1), gekennzeichnet durch die Kombination der folgenden drei Verfahrensschritte :
I) Aufbringen einer Oxidationsfärbemischung auf das Haar (Siehe Anspruch 1)
II) Aufbringen einer Zusammensetzung, enthaltend Komponenten, die die Reaktivität der zuerst aufgebrachten Oxidationsfärbemischung herabsetzen (Säuren, Seite 3, Zeile 12)
III) Spülen des Haares (Siehe Anspruch 1).
   Siehe auch Beispiele 1-6 (mit Natriumsulfit, Phosphorsäure).

Die erfindungsgemäße Aufgabe bestand daher darin ein Haarfärbeverfahren zu entwickeln, bei dem die Haare unter Vermeidung der oben angesprochenen Nachteile schonend oxidativ gefärbt werden können. Dabei sollte durch das Verfahren insbesondere die Schädigung gesunder, und/oder die weitere Schädigung bereits vorgeschädigter Haare durch die oxidative Behandlung minimiert und ein zufriedenstellendes Egalisierungsvermögen bei einer Folgefärbung erzielt werden.

Ein solches Verfahren eignet sich insbesondere für Personen, die sich in regelmäßigen Zeitabständen das Haar in der gleichen oder einer ähnlichen Nuance nachfärben, da der frisch nachgewachsene Haaransatz auf den gewünschten Farbton gebracht und die durch

Haarewaschen und Witterungsbedingungen bedingten Farbstoffverluste gleichzeitig ausgeglichen werden können.

Überraschenderweise konnte ein Verfahren gefunden werden, das unter Zuhilfenahme spezieller Mittel diesen Anforderungen in hohem Maße gerecht wird.

Gegenstand der Erfindung ist daher ein Verfahren zur schonenden Färbung und/oder Nachfärbung von menschlichen Haaren, das die folgenden drei Verfahrensschritte umfasst:
I) Aufbringen einer Oxidationsfärbemischung auf das Haar - bei Folgeanwendungen insbesondere auf den nachgewachsenen, noch ungefärbten Haaransatz;
II) Aufbringen einer Zusammensetzung, enthaltend Komponenten, die die Reaktivität der zuerst aufgebrachten Oxidationsfärbemischung herabsetzen ausgewählt aus Triethylcitrat, Diethylcitrat und/oder Monoethylcitrat;
III) Spülen des Haares.

Erfindungsgemäß bevorzugt ist ein Verfahrensschritt II), bei dem insbesondere der Längen- und Spitzenbereich der Haare behandelt wird.

Erfindungsgemäß bevorzugt ist ein Verfahren, bei dem die im Schritt I) verwendete Oxidationsfärbemischung einen alkalischen pH-Wert im Bereich von 8 bis 11 und die im Schritt II) verwendete Haarfärbezubereitung einen pH-Wert im schwach sauren bis neutralen Bereich von 4 bis 7 aufweist.

Das Oxidationsfärbemittel kann prinzipiell jedes im Stand der Technik bekannte oder in der Praxis übliche Oxidationsfärbemittel sein. Solche Zubereitungen enthalten Oxidationsfarbstoffvorprodukte in einem wässrigen Träger und werden unmittelbar vor der Anwendung mit einer Oxidationsmittelzubereitung vermischt. In dem dabei gebildeten Färbeansatz wird durch das Oxidationsmittel eine Oxidation der sogenannten Entwicklerverbindungen zu polymerisierbaren Zwischenstufen eingeleitet, die durch Polymerisation - gegebenenfalls unter Einbau der sogenannten Kupplerverbindungen (colour modifier) - zum eigentlichen Farbstoff abreagieren.

In der Regel gelingt es nicht, allein mit einer Entwicklerkomponente eine auf dem Haar natürlich wirkende Farbnuance zu erhalten. In der Praxis werden daher meist Kombinationen mit Kupplerkomponenten und mit anderen Entwicklerkomponenten eingesetzt.

Die als Oxidationsfärbemittelvorprodukte bekannten Entwicklerverbindungen sind üblicherweise primäre aromatische Amine, die wenigstens eine weitere, in para- oder ortho-Position befindliche freie oder substituierte Amino-oder Hydroxylgruppe aufweisen. Weitere bekannte Entwickler sind heterocyclische Hydrazone, 4-Aminopyrazolon-Derivate, 2,4,5,6-Tetraaminopyrimidin und dessen Derivate und Analoga.

Erfindungsgemäß bevorzugte Entwicklerkomponenten sind p-Phenylendiamin, p-Toluylendiamin, p-Aminophenol, o-Aminophenol, 1-(2'-Hydroxyethyl)-2,5-diaminobenzol, N,N-Bis-(2-hydroxy-ethyl)-p-phenylendiamin, 2-(2,5-Diaminophenoxy)-ethanol, 4-Amino-3-methylphenol, 2,4,5,6-Tetraaminopyrimidin, 2-Hydroxy-4,5,6-triaminopyrimidin, 4-Hydroxy-2,5,6-triaminopyrimidin, 2,4-Dihydroxy-5,6-diaminopyrimidin, 2-Dimethylamino-4,5,6-triaminopyrimidin, 2-Hydroxymethylamino-4-amino-phenol, 4,4'-Diaminodiphenylamin, 4-Amino-3-fluorphenol, 2-Amino-methyl-4-aminophenol, 2-Diethylaminomethyl-4-aminophenol, Bis-(2-hydroxy-5-aminophenyl)-methan, 1,4-Bis-(4-aminophenyl)-diazacycloheptan, 1,3-Bis(N(2-hydroxyethyl)-N(4-aminophenylamino))-2-propanol, 4-Amino-2-(2-hydroxyethoxy)-phenol sowie 4,5-Diaminopyrazol-Derivate nach EP 0 740 931 bzw. WO 94/08970 wie z. B. 4,5-Diamino-1-(2' -hydroxyethyl)-pyrazol.

Besonders bevorzugte Entwicklerkomponenten sind p-Phenylendiamin, p-Toluylendiamin, p-Aminophenol, 1-(2'-Hydroxyethyl)-2,5-diaminobenzol, 4-Amino-3-methylphenol, 2-Aminomethyl-4-aminophenol, 2,4,5,6-Tetraaminopyrimidin, 2-Hydroxy-4,5,6-triamino-primidin, 4-Hydroxy-2,5,6-triaminopyrimidin, Bis-(2-hydroxy-5-aminophenyl)-methan sowie 4,5-Diamino-1-(2'-hydroxyethyl)pyrazol.

Zur Nuancierung der erzielbaren Farbtöne können die erfindungsgemäßen Mittel weiterhin noch eine oder mehrere Kupplerkomponenten enthalten. Kupplersubstanzen sind häufig aromatische oder heterocyclische Ringsysteme, die zwei reaktive Gruppen in meta-Stellung aufweisen. Als Kupplerkomponenten werden in der Regel m-Phenylendiaminderivate, Naphthole, Resorcin und Resorcinderivate, Pyrazolone und m-Aminophenolderivate verwendet.

Erfindungsgemäß bevorzugte Kupplerkomponenten sind 1-Naphthol, Pyrogallol, 1,5-, 2,7- und 1,7-Dihydroxynaphthalin, o-Aminophenol, 5-Amino-2-methylphenol, m-Aminophenol, Resorcin, Resorcinmonomethylether, m-Phenylendiamin, 1-Phenyl-3-methyl-pyrazolon-5, 2,4-Dichlor-3-aminophenol, 1,3-Bis-(2,4-diaminophenoxy)-propan, 4-Chlorresorcin, 2-Chlor-6-methyl-3-aminophenol, 2-Methylresorcin, 5-Methylresorcin, 2,5-Dimethylresorcin, 2,6-Dihydroxypyridin, 2,6-Diaminopyridin, 2-Amino-3-hydroxypyridin, 2,6-Dihydroxy-3,4-diaminopyridin, 3-Amino-2-methylamino-6-methoxypyridin, 4-Amino-2-hydroxytoluol, 2,6-Bis-(2-hydroxyethylamino)-toluol, 2,4-Diaminophenoxyethanol, 1-Methoxy-2-amino-4-(2-hydroxyethylamino)-benzol, 2-Methyl-4-chlor-5-amino-phenol, 6-Methyl-1,2,3,4-tetrahydro-chinoxalin, 3,4-Methylendioxyphenol, 3,4-Methylendioxyanilin, 2,6-Dimethyl-3-amino-phenol, 2-Hydroxy-4-aminophenoxyethanol, 2-Methyl-5-(2-hydroxyethylamino)-phenol und 2,6-Dihydroxy-3,4-dimethylpyridin.

Besonders bevorzugte Kuppler-Komponenten sind 1-Naphthol, 1,5-, 2,7- und 1,7-Dihydroxynaphthalin, 5-Amino-2-methylphenol, Resorcin, 3-Aminophenol, 4-Chlorresorcin, 2-Chlor-6-methyl-3-aminophenol, 2-Methylresorcin, 5-Methylresorcin, 2,5-Dimethylresorcin, 2,6-Dihydroxy-3,4-diaminopyridin, 2,4-Diaminophenoxyethanol, 2,4-Dichlor-3-aminophenol, 2-Amino-4-hydroxyethylaminoanisol, 4-Amino-2-hydroxytoluol, 2-Amino-3-hydroxypyridin sowie 1,3-Bis-(2',4'-diaminophenoxy)-propan.

Die Entwickler- und Kupplerkomponenten werden üblicherweise in freier Form eingesetzt. Bei Substanzen mit Aminogruppen kann es aber bevorzugt sein, sie in Salzform, insbesondere in Form der Hydrochloride und Sulfate, einzusetzen.

Die erfindungsgemäßen Haarfärbemittel enthalten sowohl die Entwicklerkomponenten als auch die Kupplerkomponenten bevorzugt in einer Menge von 0,005 bis 20 Gew.-%, vorzugsweise 0,1 bis 5 Gew.-%, jeweils bezogen auf das gesamte Oxidationsfärbemittel. Üblicherweise werden Entwicklerkomponenten und Kupplerkomponenten in etwa gleichen molaren Mengen zueinander eingesetzt. Wenn sich auch der äquimolare Einsatz als zweckmäßig erwiesen hat, so ist ein gewisser Überschuss einzelner Oxidationsfarbstoffvorprodukte nicht nachteilig, so dass Entwicklerkomponenten und Kupplerkomponenten bevorzugt in einem Mol-Verhältnis von 1 : 0,5 bis 1 : 2 im Färbemittel enthalten sein können. Die Gesamtmenge an Oxidationsfarbstoffvorprodukten liegt in der Regel bei höchstens 20 Gew.-%, bezogen auf das gesamte Mittel.

Gemäß einer weiteren bevorzugten Ausführungsform des Gegenstandes der vorliegenden Erfindung kann das Farbstoffvorprodukt ein Derivat des Indolins der Formel (Ia) sein, 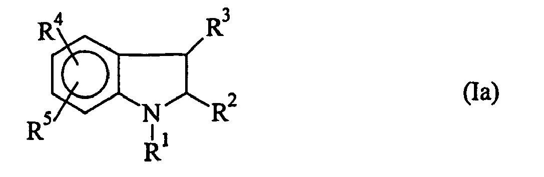 in der unabhängig voneinander R¹ steht für Wasserstoff, eine C₁- bis C₄-Alkylgruppe oder eine C₁- bis C₄-Hydroxy-alkylgruppe, R² steht für Wasserstoff oder eine -COOH-Gruppe, wobei die -COOH-Gruppe auch als Salz mit einem physiologisch verträglichen Kation vorliegen kann, R³ steht für Wasserstoff oder eine C₁- bis C₄-Alkylgruppe, R⁴ steht für Wasserstoff, eine Hydroxygruppe, eine Aminogruppe, eine C₁- bis C₄-Alkoxygruppe oder eine Gruppe -OCO-R⁶, in der R⁶ steht für eine C₁- bis C₄-Alkylgruppe, und R⁵ steht für eine der unter R⁴ genannten Gruppen, oder ein physiologisch verträgliches Salz dieser Verbindungen mit einer organischen oder anorganischen Säure, mit der Maßgabe, dass R⁴ und R⁵ nicht gleichzeitig Wasserstoff sind.

In einer dritten bevorzugten Ausführungsform dieses Gegenstandes der vorliegenden Erfindung kann das Farbstoffvorprodukt ein Derivat des Indols der Formel (Ib) sein, 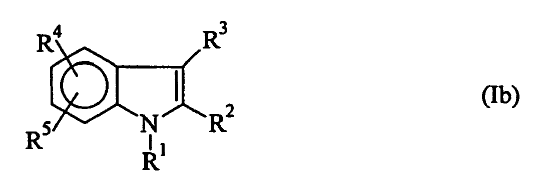 in der unabhängig voneinander R¹ steht für Wasserstoff, eine C₁- bis C₄-Alkylgruppe oder eine C₁- bis C₄-Hydroxy-alkylgruppe, R² steht für Wasserstoff oder eine -COOH-Gruppe, wobei die -COOH-Gruppe auch als Salz mit einem physiologisch verträglichen Kation vorliegen kann, R³ steht für Wasserstoff oder eine C₁- bis C₄-Alkylgruppe, R⁴ steht für Wasserstoff, eine Hydroxygruppe, eine Aminogruppe, eine C₁- bis C₄-Alkoxygruppe oder eine Gruppe -OCO-R⁶, in der R⁶ steht für eine C₁- bis C₄-Alkylgruppe, und R⁵ steht für eine der unter R⁴ genannten Gruppen, oder ein physiologisch verträgliches Salz dieser Verbindungen mit einer organischen oder anorganischen Säure, mit der Maßgabe, dass R⁴ und R⁵ nicht gleichzeitig Wasserstoff sind.

Bevorzugte Stoffe der Formel (Ia) sind 5,6-Dihydroxyindolin, N-Methyl-5,6-dihydroxyindolin, N-Ethyl-5,6-dihydroxyindolin, N-Propyl-5,6-dihydroxyindolin, N-Butyl-5,6-dihydroxyindolin, 5,6-Dihydroxyindolin-2-carbonsäure, 6-Hydroxyindolin, 6-Aminoindolin und 4-Aminoindolin. Bevorzugte Stoffe der Formel (Ib) sind 5,6-Dihydroxyindol, N-Methyl-5,6-dihydroxyindol, N-Ethyl-5,6-dihydroxyindol, N-Propyl-5,6-dihydroxyindol, N-Butyl-5,6-dihydroxyindol, 5,6-Dihydroxyindol-2-carbonsäure, 6-Hydroxyindol, 6-Aminoindol und 4-Aminoindol.

Ganz besonders bevorzugt sind 5,6-Dihydroxyindol sowie 5,6-Dihydroxyindolin in Form ihrer mineralsauren Salze.

In einer ersten bevorzugten Variante der oben beschriebenen Ausführungsformen werden die Mittel derart formuliert, dass sie als Farbstoffvorprodukte nur Indol- und/oder Indolinderivate der Formeln (Ia) und (Ib) enthalten und frei sind von üblichen Oxidationsfarbstoffvorprodukten vom Entwickler- bzw. Kupplertyp.

In einer zweiten bevorzugten Variante der oben beschriebenen Ausführungsformen können die erfindungsgemäßen Mittel neben den Indol- und/oder Indolinderivaten der Formeln (Ia) und (Ib) auch noch übliche Oxidationsfarbstoffvorprodukte vom Entwickler- bzw. Kupplertyp enthalten.

Es kann erfindungsgemäß besonders bevorzugt sein, die Indol- und/oder die Indolinderivate der Formeln (Ia) und (Ib) in Kombination mit einer oder mehrere Kupplerkomponenten in Haarfärbemitteln einzusetzen. Beispielhaft sei an dieser Stelle ausdrücklich auf die oben genannten Kupplerkomponenten verwiesen.

Bezüglich der in den erfindungsgemäßen Haarfärbe- und -tönungsmitteln einsetzbaren Farbstoffe wird weiterhin ausdrücklich auf die Monographie Ch. Zviak, The Science of Hair Care, Kapitel 7 (Seiten 248 - 250; direktziehende Farbstoffe) sowie Kapitel 8, Seiten 264 - 267 (Oxidationsfarbstoffvorprodukte), erschienen als Band 7 der Reihe "Dermatology" (Hrg.: Ch. Culnan und H. Maibach), Verlag Marcel Dekker Inc., New York, Basel, 1986, sowie das "Europäische Inventar der Kosmetik-Rohstoffe", herausgegeben von der Europäischen Gemeinschaft, erhältlich in Diskettenform vom Bundesverband Deutscher Industrie- und Handelsunternehmen für Arzneimittel, Reformwaren und Körperpflegemittel e.V., Mannheim, Bezug genommen.

Die meisten Oxidationsfärbemittel benötigen zur Einleitung der Oxidation und zur Ausbildung des Farbstoffs in einem für die Haarfärbung tragbaren Zeitraum ein chemisches Oxidationsmittel. Unter diesem Begriff soll im Rahmen dieser Erfindung ein Stoff verstanden werden, der (auch in Abwesenheit von Luftsauerstoff) die oxidative Entwicklung der Oxidationsfärbung einleiten kann. Beispiele für solche Stoffe sind Wasserstoffperoxid und dessen Anlagerungsprodukte an z. B. Harnstoff, Polyvinylpyrrolidon oder Melanin, Salze der Peroxydischwefelsäure, Perborate, Percarbonate, Perjodate, Permanganate, Hypochlorite, Chlorite, Eisen (III)-salze, Bichromat, Silberoxid, organische Percarbonsäuren und organische Peroxide.

Die Oxidationsfarbstoff-Vorprodukte werden in einen geeigneten Träger zur Aufbringung auf das Haar eingearbeitet. Solche geeigneten Träger sind für das erfindungsgemäße Verfahren bevorzugt Emulsionen und Gele.

Auch die chemischen Oxidationsmittel werden in geeignete Träger, für wasserlösliche Produkte bevorzugt Emulsionen oder Gele, eingearbeitet. Pulverförmige Produkte können auch zusammen mit inerten Trägerpulvern, z. B. mit Kieselsäure, Stärkepulver oder Holzmehl, formuliert werden.

Vor der Anwendung werden die Zubereitung der Oxidationsfarbstoff-Vorprodukte und die Zubereitung der Oxidationsmittel miteinander gemischt und der dabei gebildete Färbeansatz auf den Haaransatz aufgetragen. Hierzu eignet sich bevorzugt eine Applikationsbürste oder ein saugfähiges Substrat (Filz oder Schwamm), das für die Auftragung eines wässrigen Präparats auf Teilbereiche des Haars geeignet ist.

Nach einer Einwirkungszeit, von 5-45 Minuten, bevorzugt 10 bis 20 Minuten, erfolgt der zweite Schritt des erfindungsgemäßen Haarfärbeverfahrens.

In einer Ausführungsform des erfindungsgemäßen Haarfärbeverfahrens stehen dafür verschiedene nichtoxidative Färbesysteme zur Verfügung, die für die Entwicklung einer Färbung kein chemisches Oxidationsmittel benötigen. Hier sind zunächst die direktziehenden Färbemittel zu nennen, die entweder natürlich vorkommende Pflanzenfarbstoffe (Henna, Reng, Kamille, Walnußblätter, Krapp, Indigo) und deren Gemische oder synthetische Direktzieher umfassen.

Als synthetische Direktzieher sind vor allem die Nitroaromaten zu nennen, z. B. Nitrophenylendiamine und deren Derivate, Nitroaminophenole und deren Derivate, Azofarbstoffe, Chinolin- und Chinonimin-Farbstoffe, Triphenylmethan-, Xanthen-, Indigo- und Anthrachinon-Farbstoffe und Gemische dieser Farbstoffe. Bezüglich weiterer direktziehender Haarfarbstoffe wird wieder auf die obengenannte Monografie von Ch. Zviak und das Europäische Inventar der Kosmetik-Rohstoffe Bezug genommen.

Eine weitere Gruppe von nichtoxidativen Färbemitteln sind Systeme, deren Farbbildung durch Reaktion wenigstens zweier Komponenten ohne Beteiligung chemischer Oxidationsmittel erfolgt. So ist z. B. aus EP 0847 749 A1 bekannt, dass Derivate das Diimino-isoindolins durch Reaktion mit primären Aminen auch in Abwesenheit von Oxidationsmitteln Haarfärbungen erzeugen. In der deutschen Patentanmeldung DE 198 42 071 sind stabile Diazoniumsalze beschrieben, die durch Kupplung mit aromatischen Aminen, stickstoffhaltigen Heterozyklischen Verbindungen, Phenolen oder CH-aktiven Verbindungen interessante Haarfärbungen ergeben.

Geeignete stabile Diazoniumsalze sind z. B. 4-Diazo-diphenylaminsulfat (Variaminblausalz RT), 4-Diazo-4'-methoxydiphenylaminchlorid (Variaminblausalz B), 4-Diazo-3,2'-dimethylazo-benzolsulfat (Echtgranatsalz GBC), 2-Methoxy-4-nitrobenzoldiazonium-naphthalin-1,5-disulfat (Echtrotsalz B), 4-Diazo-2,5-dimethoxy-4'-nitroazobenzolchlorid-zinkchlorid-Doppelsalz (Echtschwarzsalz K), 2,5-Dimethoxy-4-benzoylaminophenyl-diazoniumchlorid (Echtblausalz RR), 2-Methoxy-4-nitrobenzoldiazonium-1,5-naphthalindisulfonat, 4-Chlor-2-nitrobenzoldiazoniumchlorid-zinkchlorid-Doppelsalz (Echtrotsalz 3 GL), 5-Chlor-2-methoxybenzoldiazonium-chlorid-zinkchlorid-Doppelsalz (Echtrotsalz RC), 4-Benzamido-2-methoxy-5-methylbenzoldiazonium-chlorid-zinkclorid-Doppelsalz (Echtviolettsalz B), 2-diazo-1-naphthol-4-sulfonsäure, Na-Salz der 2-Diazo-1-naphthol-5-sulfonsäure und beliebige Gemische der voranstehenden.

Eine besonders interessante Gruppe von Farbstoffen, deren Ausbildung keines chemischen Oxidationsmittels bedarf, für die Ausführung der zweiten Stufe des erfindungsgemäßen Verfahrens sind die Haarfarbstoffe vom Typ der reaktiven Carbonylverbindungen. Solche Farbstoffe sind seit längerem bekannt:

Geeignete Verbindungen vom Typ der aromatischen Aldehyde sind z. B. in der Offenlegungsschriften DE 196 30 274 A1 und DE 196 30 275 A1 beschrieben. Geeignete Verbindungen sind z. B. der 2-Hydroxybenzaldehyd, der 4-Hydroxy-3-methoxybenzaldehyd (Vanillin) und der 4-Hydroxy-3-methoxy-cinnamaldehyd (Coniferylaldehyd). Weitere geeignete Arylaldehyde sind aus US 5,199,954 bekannt.

Geeignete Verbindungen vom Typ der heteroaromatischen Aldehyde sind z. B. in der deutschen Patentanmeldung DE 197 172 80.6 beschrieben. Besonders gut geeignete Farbstoffe für das erfindungsgemäße Verfahren sind z. B. trans-β-(2-Furyl)-acrolein, 1-Methylindol-3-aldehyd, 2-(1,3,3-Trimethyl-2-indolinyliden)-acetaldehyd oder Antipyrin-4-aldehyd. Spezielle Produkte dieses Typs mit einer Pyridinium-Gruppe sind in der deutschen Patentanmeldung DE 197 45 356.2 beschrieben, z. B. die sehr gut geeigneten 4-Formyl-1-methylpyridinium-benzolsulfonat und 4-Formyl-1-methylchinolinium-methan-sulfonat bzw. -methylsulfat bzw. -p-toluolsulfonat sowie das 2-Formyl-1-methylchinolinium-p-toluolsulfonat-hydrat.

Geeignete Farbstoffe vom Typ der ungesättigten Aldehyde sind z. B. in der deutschen Patentanmeldung DE 197 17 224.5 beschrieben. Für die vorliegende Erfindung eignet sich besonders gut der Glutaconaldehyd in Form seiner Salze, z. B. seines Alkali- oder Tetrabutylammoniumsalzes oder der 2-Chlor-3-hydroxymethylen-1-cyclohexen-1-aldehyd.

Dialdehyde und Diketone und deren Derivate, die sich als Farbstoffe für das erfindungsgemäße Verfahren eignen, sind z. B. cyclische 1,2- und 1,3-Dicarbonyl-verbindungen, wie Isatin, Ninhydrin, Alloxan, Isobarbitursäure, p- und o-Chinone, 1,3-Indandione und deren Derivate. Solche Farbstoffe finden sich z. B. in der deutschen Offenlegungsschrift DE 43 35 627 A1. Geeignete Verbindungen sind z. B. der Malon-dialdehyd, bevorzugt in Form seines Dimethylacetals, das 2-Nitro-1,3-indandion oder das 2-Acetyl-1,3-cyclohexandion. Eine geeignete 1,2-Dicarbonylverbindung ist auch das Isatinsäure-Kaliumsalz.

Zu den erfindungsgemäß geeigneten Diketonen gehören auch cyclische Dicarbonylverbindungen wie z. B. das Isatin und dessen Derivate, wie sie z. B. in der deutschen Offenlegungsschrift DE 44 09 143 A1 beschrieben sind. Für das erfindungsgemäße Verfahren sind z. B. das Isatin-5-Sulfonsäure-Kaliumsalz, das N-Allylisatin, das 1-Piperidinomethylisatin, das 1-Hydroxymethylisatin und das 1-Diethylaminomethylisatin geeignet.

Eine weitere geeignete cyclische Dicarbonylverbindung ist z. B. auch die Dehydroascorbinsäure, deren Eignung als Haarfarbstoff aus der deutschen Patentanmeldung DE 197 45 354.6 bekannt ist. Schließlich eignen sich auch die Acetale, Iminderivate und Halbaminale der genannten reaktiven Carbonylverbindungen. Solche Verbindungen werden durch Reaktion der Carboxylgruppe mit primären Alkoholen oder Aminen und ggf. Wasserabspaltung erhalten.

Ausgehend von den ungesättigten Dialdehyden und Diketonen gelangt man dabei in die Gruppe der Merocyanin- und Cyanin- bzw. Azomethin-Farbstoffe. Geeignete IminDerivate des Glutacondialdehyds sind z. B. das Mono-N-methylanilin-Derivat des Glutaconaldehyds (5-N-Methylanilinopentadienal) oder das N-(5-Anilino-2,4-pentadien-1-yliden)-anilinium-chlorid. Ein weiterer geeigneter vinyloger Cyaninfarbstoff ist das 7-Dimethylamino-2,4,6-heptatrienyliden-dimethylammonium-perchlorat. Solche Verbindungen sind als Haarfärbe-mittel-Komponenten z. B. aus der deutschen Patentanmeldung DE 197 17 223.7 bekannt.

Viele der genannten reaktiven Carbonylverbindungen färben keratinhaltige Fasern unter Ausprägung verschiedenster Farbnuancen erst in Kombination mit einer oder mehreren Verbindungen mit primärer oder sekundärer Amino- oder Hydroxygruppe, ausgewählt aus der Gruppe der Aminosäuren und Peptide, der aromatischen Amine, Phenole, Aminophenole, stickstoffhaltigen Heterocyclen sowie CH-aktiven Verbindungen.

### Dabei werden in vielen Fällen auch dunklere Nuancen erhalten.

Geeignete Aminosäuren sind z. B. die natürlich vorkommenden und synthetischen Aminosäuren, z. B. Arginin, Histidin, Phenylalanin, Dihydroxyphenylalanin, Ornithin, Lysin. Geeignete Peptide sind vor allem Oligo- und Polypeptide, die eine ausreichende Wasserlöslichkeit in den erfindungsgemäßen Zubereitungen zur Keratinreduktion aufweisen. Als Beispiele sind z. B. Glutathion oder die in den Hydrolysaten von Kollagen, Keratin, Elastin, Casein, Pflanzenproteinen wie Sojaprotein, Weizengluten, Algenprotein oder Mandelprotein enthaltenen Oligopeptide zu nennen.

Geeignete aromatische Amine und Aminophenole sind N,N-Dimethyl-, N,N-Diethyl-, N-(2-Hydroxyethyl)-N-ethyl-, N,N-Bis-(2-hydroxyethyl)-, N-(2-Methoxyethyl-), 2-Chlor-, 2,3-, 2,4- und 2,5-Dichlor-p-phenylendiamin, 2,5-Dihydroxy-4-morpholinoanilin-dihydro-bromid, 2-, 3- und 4-Aminophenol, 2-Aminomethyl-4-aminophenol, 2-Hydroxymethyl-4-aminophenol, o- und p-Phenylendiamin, o- und m-Toluylendiamin, 2,5-Diamino-phenol, -toluol und -phenethol, 4-Amino-3-methylphenol, 2-(2,5-Diaminophenyl)-ethanol, 2,4-Diaminophenoxy-ethanol, 2-(2,5-Diaminophenoxy)-ethanol, 4-Methylamino-, 3-Amino-4-(2'hydroxyethyloxy)-, 3,4-Methylendiamino- und 3,4-Methylendioxyanilin, 3-Amino-2,4-dichlor-, 4-Methylamino-, 2-Methyl-5-amino-, 3-Methyl-4-amino-, 2-Methyl-5-(2-hydro-xyethylamino)-, 6-Methyl-3-amino-2-chlor-, 2-Methyl-5-amino-4-chlor-, 3,4-Methylen-dioxy-, 5-(2-Hydroxyethylamino)-4-methoxy-2-methyl- und 4-Amino-2-hydroxymethyl-phenol, 1,3-Diamino-2,4-dimethoxybenzol, 2-, 3-, 4-Aminobenzoesäure, -phenylessig-säure, 2,3-, 2,4-, 2,5-, 3,4-, 3,5-Diaminobenzoesäure, 4-, 5-Aminosalicylsäure, 3-Amino-4-hydroxy-, 4-Amino-3-hydroxy-benzoesäure, 2-, 3-, 4-Aminobenzolsulfonsäure, 3-Amino-4-hydroxybenzolsulfonsäure, 4-Amino-3-hydroxynaphthalin-1-sulfonsäure, 6-Amino-7-hydroxynaphthalin-2-sulfonsäure, 7-Amino-4-hydroxynaphthalin-2-sulfonsäure, 4-Amino-5-hydroxynaphthalin-2,7-disulfonsäure, 3-Amino-2-naphthoesäure, 3-Aminophthalsäure, 5-Aminoisophthalsäure, 1,3,5-, 1,2,4-Triaminobenzol, 1,2,4,5-Tetraaminobenzol, 2,4,5-Triaminophenol, Pentaaminobenzol, Hexaaminobenzol, 2,4,6-Triaminoresorcin, 4,5-Diaminobrenzcatechin, 4,6-Diaminopyrogallol, 3,5-Diamino-4-hydroxybrenzcatechin, aromatische Aniline bzw. Phenole mit einem weiteren aromatischen Rest, wie sie in der Formel I dargestellt sind 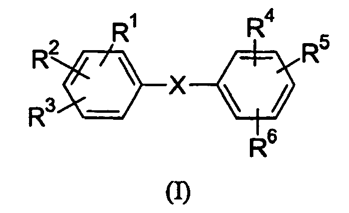 in der R¹ für eine Hydroxy- oder eine Aminogruppe, die durch C₁₋₄-Alkyl, C₁₋₄-Hydroxyalkyl- oder C₁₋₄-Alkoxy-C₁₋₄-alkyl substituiert sein kann, steht, R², R³, R⁴, R⁵ und R⁶ für Wasserstoff, eine Hydroxy- oder eine Aminogruppe, die durch C₁₋₄-Alkyl-, C₁₋₄-Hydroxyalkyl, C₁₋₄-Aminoalkyl- oder C₁₋₄-Alkoxy-C₁₋₄-alkylgruppe substituiert sein kann, für eine Carbon- oder Sulfonsäuregruppe stehen, und X für eine direkte Bindung, eine gesättigte oder ungesättigte, ggf. durch Hydroxygruppen substituierte Kohlenstoffkette mit 1 bis 4 Kohlenstoffatomen, eine Carbonyl-, Sulfonyl- oder Iminogruppe, ein Sauerstoff- oder Schwefelatom, oder eine Gruppe mit der Formel II

Z-(CH₂-Y-CH₂-Z')o (II),

in der Y eine direkte Bindung, eine CH₂- oder CHOH-Gruppe bedeutet,
Z und Z' unabhängig voneinander ein Sauerstoffatom, eine NR⁷-Gruppe, worin R⁷ Wasserstoff, eine C₁₋₄-Alkyl- oder Hydroxy-C₁₋₄-alkylgruppe bedeutet, die Gruppe -O-(CH₂)ₚ-NH oder NH-(CH₂)ₚ-O, worin p und p' 2 oder 3 sind, stehen und
o eine Zahl von 1 bis 4 bedeutet,
wie beispielsweise 4,4'-Diaminostilben, 4,4'-Diaminostilben-2,2'-disulfonsäure-mono- oder -di-Na-Salz, 4,4'-Diaminodiphenylmethan, -sulfid, -sulfoxid, -amin, 4,4'-Diaminodiphenyl-amin-2-sulfonsäure, 4,4'-Diaminobenzophenon, -diphenylether, 3,3',4,4'-Tetraaminodi-phenyl, 3,3',4,4'-Tetraamino-benzophenon, 1,3-Bis-(2,4-diaminophenoxy)-propan, 1,8-Bis-(2,5-diaminophenoxy)-3,6-dioxaoctan, 1,3-Bis-(4-aminophenylamino)-propan, -2-propa-nol, 1,3-Bis-[N-(4-aminophenyl)-2-hydroxyethylamino]-2-propanol, N,N-Bis-[2-(4-amino-phenoxy)-ethyl]-methylamin, N-Phenyl-1,4-phenylendiamin.

Die vorgenannten Verbindungen können sowohl in freier Form als auch in Form ihrer physiologisch verträglichen Salze, insbesondere als Salze anorganischer Säuren, wie Salz- oder Schwefelsäure, eingesetzt werden.

Geeignete Phenole sind z. B. das 2-, 3- oder 4-Methoxy-, das 3-Dimethylamino-, 2-(2-Hydroxyethyl)- und das 3,4-Methylendioxy-phenol, das Resorcin und das 2-, 4- und 5-Methylresorcin, das 2- und 4-Chlorresorcin, 2,5-Dimethylresorcin, Brenzkatechin, Hydrochinon, Pyrogallol, Phloroglucin, Hydroxyhydrochinon, die 2,4- oder 3,4-Dihydroxybenzoe- oder -phenylessigsäure, die Gallussäure, die 2,4,6-Trihydroxybenzoesäure oder das 2,4,5-Trihydroxyacetophenon, das 1-Naphthol, das 1,5-, 2,3- und 2,7-Dihydroxynaphthalin, die 6-Dimethylamino-4-hydroxy-2-naphthalinsulfonsäure oder die 3,6-Dihydroxy-2,7-naphthalindisulfonsäure.

Geeignete stickstoffhaltige heterocyclische Verbindungen sind z. B. 2-, 3-, 4-Amino-, 2-Amino-3-hydroxy-, 2,6-Diamino-, 2,5-Diamino-, 2,3-Diamino-, 2-Dimethylamino-5-amino-, 2-Methylamino-3-amino-6-methoxy-, 2,3-Diamino-6-methoxy-, 2,6-Dimethoxy-3,5-diamino-, 2,4,5-Triamino- und 2,6-Dihydroxy-3,4-dimethylpyridin, 2,4-Dihydroxy-5,6-diamino-, 4,5,6-Triamino-, 4-Hydroxy-2,5,6-triamino-, 2-Hydroxy-4,5,6-triamino-, 2,4,5,6-Tetraamino-, 2-Methylamino-4,5,6-triamino-, 2,4-, 4,5-Diamino- und 2-Amino-4-methoxy-6-methyl-pyrimidin, 3-Amino-, 3-Amino-5-hydroxy- und 3,5-Diaminopyrazol, 2-,3-, 8-Aminochinolin, 4-Amino-chinaldin, 2-, 6-Aminonicotinsäure, 5-Aminoisochinolin, 5-, 6-Aminoindazol, 5- und 7-Amino-benzimidazol und -benzothiazol, 2,5-Dihydroxy-4-morpholinoanilin sowie Indol- und Indolinderivate, wie 4-, 5-, 6- und 7-Aminoindol, 5,6-Dihydroxyindol, 5,6-Dihydroxyindolin und 4-Hydroxyindolin. Die vorgenannten Verbindungen können sowohl in freier Form als auch in Form ihrer physiologisch verträglichen Salze, z. B. als Salze anorganischer Säuren, wie Salz- oder Schwefelsäure, eingesetzt werden.

Diese Färbesysteme können noch weiter verstärkt werden durch geeignete stickstoffhaltige Heterocyclen wie z. B. Piperidin, Piperidin-2-, -3- oder -4-carbonsäure, Pyridin, 2-, 3- oder 4-Hydroxypyridin, Imidazol, 1-Methylimidazol, Histidin, Pyrrolidin, Pyrrolidon, Pyrrolidon-5-carbonsäure, Pyrazol, 1,2,4-Triazol, Piperazin sowie deren physiologisch verträgliche Salze.

Auch CH-aktive Verbindungen können zur Farbentwicklung mit reaktiven Carbonylverbindungen verwendet werden. Geeignete CH-aktive Verbindungen sind z. B. 1,2,3,3-Tetramethyl-3H-indoliumiodid, 1,2,3,3-tetramethyl-3H-indolium-p-toluolsulfonat, 1,2,3,3-Tetramethyl-3H-indolium-methansulfonat, Fischersche Base (1,3,3-Trimethyl-2-methylen-indolin), 2,3-Dimethyl-benzothiazoliumiodid, 2,3-Dimethyl-benzothiazoliump-toluolsulfonat, Rhodamin, Rhodanin-3-essigsäure, 1-Ethyl-2-chinaldinium-iodid, 1,4-Dimethylchinolium-iodid, 1-Methyl-2-chinaldinium-iodid, 1,4-Dimethylchinoliniumiodid, Barbitursäure, Thiobarbitursäure, 1,3-Dimethylthiobarbitursäure, 1,3-Diethylthiobarbitursäure, Diethylthio-barbitursäure, Oxindol, 3-Indoxylacetat, Cumaranon und 1-Methyl-3-phenyl-2-parazolinon.

In der Färbemittelzubereitung für die zweite Stufe des erfindungsgemäßen Verfahrens können mehrere Farbstoffe vom Typ der reaktiven Carbonylverbindungen gleichzeitig enthalten sein. Desgleichen können auch mehrere Verbindungen aus der Gruppe der Aminosäuren, Peptide, aromatischen Amine, Aminophenole, Phenole und stickstoffhaltigen Heterocyclen gemeinsam enthalten sein, wenn dies zur Erzielung der gewünschten Farbnuance erforderlich ist.

In einer bevorzugten Ausführungsform ist das in der zweiten Stufe zur Längenfärbung verwendete Haarfärbemittel ausgewählt aus einer Gruppe, bestehend aus direktziehenden Naturfarbstoffen, direktziehenden Nitroaromaten, kationischen direktziehenden Haarfarbstoffen oder einem Färbesystem aus reaktiven Carbonylverbindungen oder Diazoniumsalzen in Kombination mit farbverstärkenden Amino- oder Hydroxylverbindungen.

Es sind auch Oxidationsfarbstoff-Vorprodukte bekannt, die ohne chemische Oxidationsmittel allein durch die Anwesenheit von Luftsauerstoff auf dem Haar Oxidationsfärbungen erzeugen können und sich daher für die Verwendung in der zweiten Stufe des erfindungsgemäßen Haarfärbeverfahrens eignen. Beispiele für solche Oxidationsfärbemittel sind z. B. in EP 0 074 243 B1 beschrieben. Weitere Beispiele sind die aus EP 0 530 229 B1 bekannten und bereits weiter oben beschriebenen IndolinDerivate der Formel Ia und Indolderivate der Formel Ib.

Diese und andere Oxidationsfarbstoff-Vorprodukte können aber auch durch bestimmte Katalysatoren zur Bildung von Oxidationsfärbungen aktiviert - oder in ihrer Farbentwicklung beschleunigt werden.

In einer weiteren, bevorzugten Ausführungsform umfasst das in der zweiten Stufe zur Längenfärbung verwendete Haarfärbemittel neben den direktziehenden Farbstoffen weiterhin ein Oxidationsfärbemittel, dessen Oxidationsfarbstoffvorprodukte in Gegenwart geeigneter Katalysatoren durch Luftsauerstoff aktiviert werden.

Katalysatoren, die eine Aktivierung der Luftoxidation bewirken, sind z. B. Übergangsmetallsalze wie z. B. Mangan-, Kupfer-, Eisen-, Ruthenium-, Kobalt-, Molybdän- und Vanadiumsalze.

Geeignete Metallkatalysatoren sind z. B. aus DE 2 222 001 A1, JP 03/258713 A2 und JP 53/072836 A2 bekannt. In einer bevorzugten Ausführungsform sind die Metalle mit mindestens einem mehrzähnigen Liganden komplexiert und in Hohlräume von Käfigverbindungen, z. B. Zeolithen, eingeschlossen.

Als Katalysatoren eignen sich auch Enzyme, z. B. Oxidasen wie z. B. Uricase, Phenoloxidasen wie z. B. Laccasen, Tyrosinasen oder Oxidoreduktasen, welche eine Oxidation der Farbstoffvorprodukte biokatalytisch unter Reduktion des entsprechenden Substrats aktivieren.

Besonders gut eignen sich sogen. 2-Elektronen-Oxidoreduktasen in Kombination mit den dafür spezifischen Substraten. Solche Systeme sind z. B.
- Pyranose-Oxidase und z. B. D-Glucose oder Galactose (als Substrat)
- Glucose-Oxidase und D-Glucose
- Glycerin-Oxidase und Glycerin
- Pyruvat-Oxidase und Brenztraubensäure (oder deren Salze)
- Alkohol-Oxidase und Alkohol (z. B. MeOH, EtOH)
- Lactat-Oxidase und Milchsäure und deren Salze
- Tyrosinase und Tyrosin
- Uricase und Harnsäure und deren Salze
- Cholinoxidase und Cholin und
- Aminosäure-Oxidase und Aminosäure.

Gut eignen sich insbesondere auch Kombinationen solcher Oxidoreduktase-Systeme mit Peroxidasen.

Enzymatisch aktivierte Oxidationshaarfärbemittel sind z. B. aus WO 94/00100 A1, EP 0 310 675 A1, EP 0 504 005 A1 und EP 0 795 313 A2 bekannt.

Um die Haare während des Färbevorgangs möglichst wenig zu schädigen, setzt man dem Färbemittel des erfindungsgemäßen Verfahrens weitere Komponenten zu, die die Reaktivität der erstaufgebrachten Färbemischung herabsetzen. Dadurch sollen die beiden Hauptursachen für die Haarschädigung während des Färbevorgangs, der alkalische pH-Wert sowie der überschüssige Anteil an Oxidationsmittel, weitgehend beseitigt werden. Insbesondere bei Folgeanwendungen kann es erfindungsgemäß weiterhin bevorzugt sein, durch die Zugabe von bestimmten Salzen im zweiten Verfahrensschritt die Viskosität der Oxidationsfärbemischung herabzusetzen, damit sich die Mischung besser im Resthaar verteilt. Die Komponenten sollten aber auch derart gewählt sein, dass sie haarstrukturierend wirken und einer zusätzlichen Schädigung vorbeugen.

Als Komponenten, die die Reaktivität der erstaufgebrachten Oxidationsfärbemischung herabsetzen, enthält die Zusammensetzung der Stufe II Triethylcitrat, Diethylcitrat und/oder Monoethylcitrat. Zusätzlich eignen sich erfindungsgemäß insbesondere
- Säuren und deren Derivate wie beispielsweise Ascorbinsäure, Isoascorbinsäure, Phosphorsäure, Zitronensäure, Gallussäure, Weinsäure, Milchsäure, die den pH-Wert der Oxidationsfärbemischung von einem Startwert von 9 bis 11 auf einen PH-Wert von 7 bis 8 senken. Besonders bevorzugt im Sinne der Erfindung ist Ascorbinsäure.
- Reduktionsmittel wie beispielsweise Ascorbinsäure, Isoascorbinsäure, Trinatriumcitrat, N-Acetyl-L-cystein und Natriumsulfit, die den überschüssigen Wasserstoffperoxidanteil in der erstaufgebrachten Oxidationsfärbemischung herabsetzen. Insbesondere Ascorbinsäure und/oder mehrwertige Alkohole wie Glycerin, tert-Butylmethoxyphenol und 2,6-Di-tert.-butyl-4-methoxyphenol sowie Enzyme, insbesondere Katalase, haben sich als erfindungsgemäß geeignet erwiesen,
- Organische und/oder anorganische Salze, insbesondere wasserlösliche Natrium-, Kalium-, Magnesium- und/oder Calciumsalze zur Herabsetzung der Viskosität,
- Haarstrukturierende Substanzen, insbesondere Kohlenhydrat-Derivate und/oder Carboxylverbindungen und/oder Vitamine, Provitamine und deren Derivate zur Stärkung der strapazierten Haarspitzen.
   Unter geeigneten Kohlenhydrat-Derivaten im Sinne der Erfindung sind zu verstehen:
   - Kohlenhydrate, Zuckeralkohole und Zucker sowie deren Salze, insbesondere Monosaccharide, Disaccharide, Trisaccharide und Oligosaccharide, wobei diese auch in Form von Aldosen, Ketosen und/oder Lactosen, sowie geschützt durch übliche und in der Literatur bekannte -OH - und -NH - Schutzgruppen, wie beispielsweise die Triflatgruppe, die Trimethylsilylgruppe oder Acylgruppen sowie weiterhin in Form der Methylether und als Phosphatester, vorliegen können,
   - Aminodesoxyzucker, Desoxyzucker, Thiozucker, wobei diese auch in Form von Aldosen, Ketosen und/oder Lactosen, sowie geschützt durch übliche und in der Literatur bekannte -OH - und -NH - Schutzgruppen, wie beispielsweise die Triflatgruppe, die Trimethylsilylgruppe oder Acylgruppen sowie weiterhin in Form der Methylether und als Phosphatester, vorliegen können.

Ganz besonders bevorzugt sind hierunter Monosaccharide mit 3 bis 8 C - Atomen, wie beispielsweise Triosen, Tetrosen, Pentosen, Hexosen, Heptosen und Octosen, wobei diese auch in Form von Aldosen, Ketosen und/oder Lactosen sowie geschützt durch übliche und in der Literatur bekannte -OH - und -NH - Schutzgruppen, wie beispielsweise die Triflatgruppe, die Trimethylsilylgruppe oder Acylgruppen sowie weiterhin in Form der Methylether und als Phosphatester, vorliegen können.
Weiterhin sind ganz besonders bevorzugt Oligosaccharide mit bis zu 50 Monomereinheiten, wobei diese auch in Form von Aldosen, Ketosen und/oder Lactosen sowie geschützt durch übliche und in der Literatur bekannte -OH - und -NH - Schutzgruppen, wie beispielsweise die Triflatgruppe, die Trimethylsilylgruppe oder Acylgruppen sowie weiterhin in Form der Methylether und als Phosphatester, vorliegen können.
Beispielhaft seien erwähnt Sorbit, Inosit, Mannit, Tetrite, Pentite, Hexite, Threit, Erythrit, Adonit, Arabit, Xylit, Dulcit, Erythrose, Threose, Arabinose, Ribose, Xylose, Lyxose, Glucose, Galactose, Mannose, Allose, Altrose, Gulose, Idose, Talose, Fructose, Sorbose, Psicose, Tegatose, Desoxyribose, Glucosamin, Galaktosamin, Rhamnose, Digitoxose, Thioglucose, Saccharose, Lactose, Trehalose, Maltose, Cellobiose, Melibiose, Gestiobiose, Rutinose, Raffinose sowie Cellotriose. Weiterhin sei auf die einschlägige Fachliteratur wie beispielsweise Beyer-Walter, Lehrbuch der organischen Chemie, S. Hirzel Verlag Stuttgart, 19. Auflage, Abschnitt III, Seiten 393 und folgende verwiesen.
Selbstverständlich umfaßt die erfindungsgemäße Lehre alle isomeren Formen, wie cis - trans - Isomere, Diastereomere, Epimere, Anomere und chirale Isomere.

Geeignete **Carboxylverbindungen** im Sinne der Erfindung sind beispielsweise:
- Ameisensäure, Essigsäure, Propionsäure, Buttersäure, Isobuttersäure, Valeriansäure, Isovaleriansäure, Pivalinsäure, Oxalsäure, Malonsäure, Bernsteinsäure, Glutarsäure, Glycerinsäure, Glyoxylsäure, Adipinsäure, Pimelinsäure, Korksäure, Azelainsäure, Sebacinsäure, Propiolsäure, Crotonsäure, Isocrotonsäure, Elaidinsäure, Maleinsäure, Fumarsäure, (N-(2,4-Dihydroxy-3,3-dimethylbutyryl)-β-alanin), Muconsäure, Citraconsäure, Mesaconsäure, Camphersäure, Benzoesäure, o,m,p-Phthalsäure, Naphthoesäure, Toluoylsäure, Hydratropasäure, Atropasäure, Zimtsäure, Isonicotinsäure, Nicotinsäure, Bicarbaminsäure, 4,4'-Dicyano-6,6'-binicotinsäure, 8-Carbamoyloctansäure, 1,2,4-Pentantricarbonsäure, 2-Pyrrolcarbonsäure, 1,2,4,6,7-Napthalinpentaessigsäure, Malonaldehydsäure, 4-Hydroxy-phthalamidsäure, 1-Pyrazolcarbonsäure, Gallussäure oder Propantricarbonsäure, eine Dicarbonsäure ausgewählt aus der Gruppe, die gebildet wird durch Verbindungen der allgemeinen Formel (I), 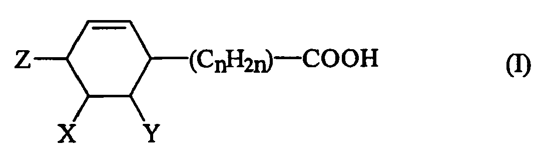 in der Z steht für eine lineare oder verzweigte Alkyl- oder Alkenylgruppe mit 4 bis 12 Kohlenstoffatomen, n für eine Zahl von 4 bis 12 sowie eine der beiden Gruppen X und Y für eine COOH-Gruppe und die andere für Wasserstoff oder einen Methyl- oder Ethylrest, Dicarbonsäuren der allgemeinen Formel (I), die zusätzlich noch 1 bis 3 Methyl- oder Ethylsubstituenten am Cyclohexenring tragen sowie Dicarbonsäuren, die aus den Dicarbonsäuren gemäß Formel (I) formal durch Anlagerung eines Moleküls Wasser an die Doppelbindung im Cyclohexenring entstehen sowie
- Hydroxycarbonsäuren und hierbei wiederum insbesondere die Dihydroxy-, Trihydroxy- und Polyhydroxycarbonsäuren sowie die Dihydroxy-, Trihydroxy- und Polyhydroxy- di-, tri- und polycarbonsäuren.

Besonders geeignete **Vitamine, Provitamine und deren Derivate** im Sinne der Erfindung sind Panthenol, Vitamin B6, Niacinamid und Pantolacton.

Die Komponenten zur Herabsetzung der Reaktivität der erstaufgebrachten Oxidationsfärbemischung werden erfindungsgemäß in Konzentrationen von 0,001 Gew.% bis 5 Gew.%, vorzugsweise von 0,05 Gew.% bis 3 Gew.-% und ganz besonders bevorzugt in Mengen von 0,1 Gew.% bis zu 2 Gew.% eingesetzt.

Das erfindungsgemäße Verfahren zur schonenden Färbung der Haare benötigt zu seiner Durchführung verschiedene Zubereitungen, die, insbesondere für die Heimanwendung, bevorzugt getrennt verpackt und zu einer Verkaufseinheit zusammengefasst sind. Dies hat den Vorteil, dass die Komponenten in den für die Anwendung optimalen Mengenverhältnissen vorbereitet und mit einer einfachen Gebrauchsanleitung ausgestattet werden können und bis zur unmittelbaren Vereinigung zum Färbeansatz getrennt bleiben können, so dass eine vorzeitige Einleitung der Farbstoffentwicklung vermieden wird.

Ein weiterer Gegenstand der Erfindung ist daher ein Kit zur Durchführung des erfindungsgemäßen Verfahrens bestehend aus den getrennt verpackten, zu einer Verkaufseinheit zusammengefassten Komponenten
a) einer Zubereitung von Oxidationsfarbstoffvorprodukten,
b) einer Oxidationsmittelzubereitung und
c) einer Zubereitung von Komponenten, die die Reaktivität der erstaufgebrachten Oxidationsfärbemischung herabsetzt, ausgewählt aus Triethylcitrat, Diethylcitrat und/oder Monoethylcitrat.
   In der Vergangenheit hat sich herausgestellt, dass der Verbraucher einfach handhabbare Produkte mit weniger Verpackungsaufwand aus praktischen und umweltökonomischen Gründen bevorzugt. Viele Komponenten der Zubereitung c), die die Reaktivität der Oxidationsfarbstoffmischung herabsetzen sollen, sind jedoch im oxidativen Medium oder unter alkalischen Bedingungen nicht stabil und mussten daher bislang in einem separaten Behälter aufbewahrt werden. Um die oben genannten Bedürfnisse dennoch zu erfüllen, stellte sich somit weiterhin die Aufgabe ein weiteres Kit und ein weiteres Färbeverfahren zu entwickeln, das mit den gleichen Komponenten, jedoch mit weniger Verpackungsaufwand und einer kleineren Anzahl von Verfahrensschritten ebenfalls eine schonende Haarfärbung gewährleistet.

Die Anwendung der erfindungsgemäßen Haarfärbemittel erfolgt bevorzugt in der Weise, dass man zunächst die Farbstoffzubereitung a) mit der Oxidationsmittelzubereitung b) vermischt und mit Hilfe eines Applikationswerkzeuges, bevorzugt einer Bürste, auf den Haaransatz, bzw. auf den der Kopfhaut benachbarten Bereich des Haars, etwa in einer Länge von 1-3 cm Länge aufträgt und 5 bis 45 Minuten einwirken lässt.

Im Anschluß wird im ersten erfindungsgemäßen Verfahren die nichtoxidative Farbstoffzubereitung c) zugesetzt und die Gesamtmischung a) + b) + c) auf die gesamte Länge des Haares verteilt. Nach 10 bis 20 Minuten Einwirkungszeit wird mit Wasser und einem Shampoo gründlich ausgespült.

Im zweiten erfindungsgemäßen Verfahren werden nach einer Einwirkungszeit von 15 bis 20 Minuten auch die Haarlängen mit dem Haarfärbemittel behandelt. Nach weiteren 15 bis 20 Minuten Einwirkungszeit werden die Haare mit Wasser und einem handelsüblichen Shampoo gründlich ausgespült.

Der Vorteil dieses Verfahrens ist darin zu sehen, dass dem Haar während der gesamten Applikationszeit Wirkstoffe zugeführt werden können, so dass sie weniger strapaziert und schonender gefärbt werden können.

Für die Auftragung des Färbeansatzes aus den Komponenten a) und b) mit einer Applikationsbürste auf den Haaransatz ist es vorteilhaft, wenn dieser Färbeansatz eine gewisse Viskosität aufweist, die ein Herablaufen des Färbemittels vom Haar oder eine Anfärbung der Kopfhaut verhindert. Aus diesem Grund eignet sich als Träger für die Oxidationsfarbstoffvorprodukte a) eine Creme oder auch ein Gel.

Erfindungsgemäß geeignete Gele enthalten bevorzugt Alkali- oder Ammoniumseifen von C₁₂-C₂₂-Fettsäuren als Gelbildner.

Erfindungsgemäß geeignete Färbecremes enthalten zur Verdickung bevorzugt emulgierte Fettkomponenten, z. B. Fettalkohole mit 12-18 C-Atomen, Fettsäurepartialgyceride oder emulgierte kosmetische Ölkomponenten wie z. B. Paraffinöle, Pflanzenöle oder synthetische Esteröle.

Darüber hinaus können solche Zubereitungen alle in der Oxidations-Haarfärberei üblichen Hilfs- und Zusatzstoffe enthalten, dazu gehören vor allem
- Tenside, insbesondere Emulgatoren
- Reduktionsmittel wie z. B. Ascorbinsäure Thioglycolsäure oder Natriumsulfit,
- Puffersubstanzen wie z. B. CNH₄)H₂ PO₄, NH₄Cl, NH₃, Alkanolamine
- organische Lösungsmittel wie z. B. Ethanol, 1,2-Propylenglycol oder Glycerin, Diethylenglycol,
- haarkosmetische Wirksubstanzen, wie z. B. Antischuppenwirkstoffe, Vitamine, Pflanzenextrakte, Ceramide, Allantoin, Panthenol, Bisabolol, Pyrrolidoncarboxylat, Lichtschutzmittel (UV-Filter), Proteine und Proteinderivate, Aminosäuren, Cholesterin, Strukturanten wie z. B. Zucker,
- wasserlösliche Verdickungsmittel wie z. B. Pflanzengummi, Xanthan-Gum, Cellulose-Derivate, Stärkeether, Polyvinylalkohol, Polyvinylpyrrolidon, Polyethylenglycole
- Komplexbildner wie z. B. EDTA, NTA oder Acetophosphonsäuren
- Duftstoffe

Die Oxidationsmittel-Zubereitung b) kann bevorzugt eine wässrige Lösung oder eine Öl-in-Wasser-Emulsion sein.
Es ist daher darauf zu achten, dass die Mischung der Oxidationsmittelzubereitung b) mit der Farbstoffzubereitung a) problemlos und mit wenig Rühraufwand möglich ist und die Viskosität des Färbeansatzes ausreichend hoch für die Ansatzfärbung bleibt.

Die folgenden Beispiele sollen den Gegenstand der Erfindung näher erläutern:

### Anwendungsbeispiele

### Material:

Es wurden 2 g schwere und 15 cm lange Humanhaarsträhnen naturweiß der Firma Kerling verwendet.
Als Referenz-Färbeprodukt diente die Marktrezeptur Poly Diadem 718 - Haselnuss; pro Haartresse wurden 10 g dieser Färbemischung verteilt.

Die Komponente c (gemäß Beschreibung und Anspruch 6) wurde in drei Messreihen mit den folgenden Zusatzstoffen versehen:

| | **Komponente** | **Konzentration** | **pH** | **Menge pro Tresse** |
|---|---|---|---|---|
| **1** | wässrige | 0,6 mol/l | 2,3 | 1,5 |
| | Ascorbinsäurelösung | | | |
| **2** | wässrige | 0,2 mol/l | 2,2 | 1,5 |
| | Zitronensäurelösung | | | |
| **3** | wässrige Triethylcitratlösung | 3%ig | 2,7 | 1,5 |
| **4** | wässrige Triethylcitratlösung + 0,5% | - | 5 | 1,5 |
| | Pyrrilidoncarbonsäure-Na- | | | |
| | Salz + 0,5% Panthenol + | | | |
| | 2,5% Propylenglycol + | | | |
| | 0,001% HC Yellow 2 | | | |

### Vorbereitung und Messung:

Zunächst wurden die Humanhaarsträhnen präpariert, um die inhomogene Haarstruktur von der Haarwurzel bis zur Spitze einer natürlichen Haarfrisur nach einmaliger Coloration mit nachgewachsenem Ansatz zu simulieren. Dazu wurden die Tressen im unteren Bereich (ca. 5 cm, Spitzenbereich) einmalig blondiert (Marktprodukt Poly Blonde Medium). Es folgte eine Coloration der Tressen im Bereich von 2 cm unter der Bündelung bis in die Spitzen mit dem Marktprodukt Poly Diadem 718 (Erstfärbung in Längen und Spitzen mit 30-minütiger Einwirkungszeit). Anschließend wurden die Tressen 8 mal gewaschen (Nachstellung der Alterung der Coloration).
Die derart präparierten Haartressen wurden dann den Folgeanwendungen mit der Marktrezeptur Poly Diadem 718, ggf. in Kombination mit den erfindungsgemäßen Komponenten wie folgt unterzogen:
**A:** Färbung der vorcolorierten Haartresse ohne Ansatzbehandlung mit Diadem 718, d.h. von Ansatz mit Spitze mit 30-minütiger Einwirkungszeit.
**B:** Klassisch: Färbung der vorcolorierten Haartresse mit Ansatzapplikation der Coloration Diadem 718 auf die noch nicht colorierte, 2 cm lange Partie unterhalb der Bündelung (Ansatz) mit 20-minütiger Einwirkungszeit. Anschlißend Verteilung auf die restlichen Längen und Spitzen mit weiteren 10 Minuten Einwirkungszeit.
**C:** Referenz: Färbung des Ansatzes mit Diadem 718 mit 20-minütiger Einwirkungszeit; anschließend Zugabe von 1,5 g destilliertem Wasser (pH 2,5) und Verteilung auf der ganzen Tresse mit weiterer 10-minütiger Einwirkungszeit.
**D** Vergleichsbeispiel: Probe c-1: Vorgehen wie unter C, jedoch wird anstatt Wasser die Lösung c-1 auf dem Haar verteilt.
**E** Vergleichsbeispiel: Probe c-2: Vorgehen wie unter C, jedoch wird anstatt Wasser die Lösung c-2 auf dem Haar verteilt.
**F:** Probe c-3: Vorgehen wie unter C, jedoch wird anstatt Wasser die Lösung c-3 auf dem Haar verteilt.
**G:** Probe c-4: Vorgehen wie unter C, jedoch wird anstatt Wasser die Lösung c-4 auf dem Haar verteilt.

Anschließend wurden farbmetrische Untersuchungen über alle Haarpartien (Bestimmung der Farbstärke FS) und Thermoanalysen der Haarlängen (Bestimmung der Keratinschmelzpunkte KS) durchgeführt.

### Ergebnisse:

Die folgenden Ergebnisse konnten ermittelt werden:

| | | **A** | **B** | **C** | **D** | **E** | **F** | **G** |
|---|---|---|---|---|---|---|---|---|
| | **Ansatz** | 100 | 100 | 100 | 100 | 100 | 100 | 100 |
| **FS** | **Längen** | 172 | 159 | 152 | 102 | 123 | 117 | 115 |
| | **Spitzen** | 227 | 198 | 170 | 143 | 149 | 135 | 126 |
| **KS Längen** | | 135,7 | 145,3 | 146,2 | 148,5 | 148,0 | 148,6 | 150,7 |

Als Maß für die Egalisierung kann eine möglichst große Übereinstimmung der Farbstärken für den Ansatz, die Längen und die Spitzen angesehen werden.

Für den Keratinschmelzpunkt gilt, dass der Wert umso höher ist, desto kräftiger das Haar, d.h. umso schonender die Haarfärbung ist.

Aus den obigen Messwerten kann man deutlich erkennen, dass ein Zusatz der Komponente c (Versuche D bis G) extreme Vorteile für die Egalisierung der Farbe hat. Während Wasser allein als Komponente c (Versuch C) nur zu einer leichten Verbesserung der Farbegalisierung führt, wird dieser Effekt durch die erfindungsgemäßen Zusätze (Versuche D bis G) erheblich gesteigert.

Man kann den Messwerten ebenfalls entnehmen, dass ein Zusatz der Komponente c zu einem signifikanten Effekt in Bezug auf die schonende Färbung bringt. So beträgt beispielsweise der Keratinschmelzpunkt einer Haartresse, die mit Wasser als Komponente c behandelt wurde noch 146,6°C, während die erfindungsgemäßen Zusätze zu einer deutlichen Erhöhung des Keratinschmelzpunktes bis zu 150°C führen (Versuche D bis G).
Eine Ganzkopfbehandlung (Versuch A) führt hingegen zu extrem höherer Schädigung in der Haarlängen.

## Patentansprüche

1. Verfahren zur schonenden Färbung und/oder Nachfärbung von menschlichen Haaren, **gekennzeichnet durch** die Kombination der folgenden drei Verfahrensschritte:
I) Aufbringen einer Oxidationsfärbemischung auf das Haar - bei Folgeanwendungen insbesondere auf den nachgewachsenen, noch ungefärbten Haaransatz;
II) Aufbringen einer Zusammensetzung, enthaltend Komponenten, die die Reaktivität der zuerst aufgebrachten Oxidationsfärbemischung herabsetzen;
III) Spülen des Haares,
wobei als Komponente, die die Reaktivität der zuerst aufgebrachten Oxidationsfärbemischung herabsetzt, Triethylcitrat, Diethylcitrat und/oder Monoethylcitrat enthalten ist.

2. Verfahren nach Anspruch 1, **dadurch gekennzeichnet, dass** die im Schritt I) verwendete Oxidationsfärbemischung einen alkalischen pH-Wert im Bereich von 8 bis 11 und die im Schritt II) verwendete Haarfärbezubereitung einen pH-Wert im schwach sauren bis neutralen Bereich von 4 bis 7 aufweist.

3. Verfahren nach einem der Ansprüche 1 oder 2, **dadurch gekennzeichnet, dass** die im Schritt II) verwendete Zusammensetzung zusätzlich einen direktziehenden Farbstoff aus der Gruppe der direktziehenden Naturfarbstoffe und/oder der direktziehenden Nitroaromaten und/oder der kationischen direktziehenden Haarfarbstoffe enthält.

4. Verfahren nach einem der Ansprüche 1 bis 3, **dadurch gekennzeichnet, dass** die im Schritt II) verwendete Zusammensetzung zusätzlich einen Farbstoff oder ein Farbstoffvorprodukt aus der Gruppe der reaktiven Carbonylverbindungen und/oder stabilen Diazoniumsalze in Kombination mit mindestens einer Amino- oder Hydroxylverbindung enthält.

5. Kit zur Durchführung eines Verfahrens nach einem der Ansprüche 1 bis 4, bestehend aus den folgenden, getrennt verpackten und zu einer Verkaufseinheit zusammengefassten Komponenten;
a) einer Zubereitung von Oxidationsfarbstoffvorprodukten,
b) einer Oxidationsmittelzubereitung,
c) einer Zubereitung von Komponenten, die die Reaktivität der erstaufgebrachten Oxidationsfärbemischung herabsetzt
wobei als Komponente, die die Reaktivität der erstaufgebrachten Oxidationsfärbemischung herabsetzt, Triethylcitrat, Diethylcitrat und/oder Monoethylcitrat enthalten ist.

## Claims

1. Method for gently dyeing and/or redyeing human hair, **characterised by** the combination of the following three process steps:
I) applying an oxidation dye mixture onto the hair - for subsequent applications especially onto the newly grown, still undyed hair roots;
II) applying a composition comprising components that decrease the reactivity of the first applied oxidation dye mixture;
III) rinsing the hair,
wherein triethyl citrate, diethyl citrate and/or monoethyl citrate is/are comprised as the component that decreases the reactivity of the first applied oxidation dye mixture.

2. Method according to Claim 1, wherein the oxidation dye mixture used in step I) exhibits an alkaline pH in the range 8 to 11 and the hair dye preparation used in step II) exhibits a pH in the weakly acidic to neutral range 4 to 7.

3. Method according to one of Claims 1 or 2, wherein the composition used in step II) comprises a substantive dye from the group of the substantive natural dyes and/or the substantive nitro-aromatics and/or the cationic substantive dyes.

4. Method according to one of Claims 1 to 3, wherein the composition used in step II) additionally comprises a dye or a dye precursor from the group of the reactive carbonyl compounds and/or stable diazonium salts in combination with at least one amino- or hydroxyl compound.

5. Kit for carrying out a method according to one of Claims 1 to 4, consisting of the following separately packed components combined into a sales unit:
a) a preparation of oxidation dye precursors,
b) a preparation of oxidants,
c) a preparation of components, which decreases the reactivity of the initially applied oxidation dye mixture
wherein triethyl citrate, diethyl citrate and/or monoethyl citrate is/are comprised as the component that decreases the reactivity of the initially applied oxidation dye mixture.

## Revendications

1. Procédé pour la teinture et/ou la reteinture des cheveux humains de type non agressif, **caractérisé par** la combinaison des trois étapes opératoires suivantes :
I) l'application d'un mélange de colorants d'oxydation sur les cheveux - dans le cas d'applications successives, en particulier à la racine des cheveux repoussés encore non teintés ;
II) l'application d'une composition contenant des composants qui diminuent la réactivité du mélange de colorants d'oxydation appliqué en premier lieu ;
III) le rinçage des cheveux ;
dans lequel, la composition contient, à titre de composant qui diminue la réactivité du mélange de colorants d'oxydation appliqué en premier lieu, du citrate de triéthyle, du citrate de diéthyle et/ou du citrate de monoéthyle.

2. Procédé selon la revendication 1, **caractérisé en ce que** le mélange de colorants d'oxydation que l'on utilise à l'étape I) présente une valeur de pH alcalin dans la plage de 8 à 11 et la préparation de teinture capillaire que l'on utilise à l'étape II) présente une valeur de pH dans la plage faiblement acide à neutre de 4 à 7.

3. Procédé selon l'une quelconque des revendications 1 ou 2, **caractérisé en ce que** la composition que l'on utilise à l'étape II) contient en outre un colorant montant directement sur la fibre choisi parmi le groupe des colorants naturels montant directement sur la fibre et/ou des composés nitroaromatiques montant directement sur la fibre et/ou des teintures capillaires de type cationique montant directement sur la fibre.

4. Procédé selon l'une quelconque des revendications 1 à 3, **caractérisé en ce que** la composition que l'on utilise à l'étape II) contient en outre un colorant ou un précurseur de colorants choisis parmi le groupe des composés carbonyle réactifs et/ou des sels stables de diazonium en combinaison avec au moins un composé amino ou un composé hydroxyle.

5. Nécessaire pour la mise en oeuvre d'un procédé selon l'une quelconque des revendications 1 à 4, constitué par les composants suivants, emballés séparément et assemblés pour former une unité de vente :
a) une préparation de précurseurs de colorants d'oxydation ;
b) une préparation d'agents d'oxydation ;
c) une préparation de composants qui diminuent la réactivité du mélange de colorants d'oxydation appliqué en premier lieu ; dans lequel, la préparation contient, à titre de composant qui diminue la réactivité du mélange de colorants d'oxydation appliqué en premier lieu, du citrate de triéthyle, du citrate de diéthyle et/ou du citrate de monoéthyle.
